# EUROPEAN PATENT SPECIFICATION

(11) **EP 2 669 306 B1**
(45) Date of publication and mention of the grant of the patent: **12.08.2015**
(21) Application number: 12170572.7
(22) Date of filing: 01.06.2012
(51) Int. Cl.: C08G 59/04, C08G 59/06, C08G 59/02, C07D 301/24

(54) **Process for manufacturing an epoxy resin**
Verfahren zur Herstellung eines Epoxidharzes
Procédé de fabrication d'une résine époxy

(43) Date of publication of application: 04.12.2013
(73) Proprietor: SOLVAY SA, 1120 Bruxelles (BE)
(72) Inventor: Krafft, Philippe, 1640 RHODE SAINT GENESE (BE); Gilbeau, Patrick, 7090 BRAINE-LE-COMTE (BE)
(74) Representative: Vande Gucht, Anne

(56) References cited:
- EP-A1- 0 082 572
- WO-A1-2011/054770
- GB-A- 859 456
- US-A- 3 138 618
- DATABASE CAPLUS [Online] CHEMICAL ABSTRACTS SERVICE, COLUMBUS, OHIO, US; Shan et al: "Method for preparing bisphenol epox resin with dichloropropanol", XP002685670, Database accession no. 2009:1086851

## Description

The present invention relates to a process for manufacturing an epoxy resin.

The present invention relates more specifically to a process for manufacturing an epoxy resin, in particular by using dichloropropanol as starting material.

Epoxy resins may be used in "coating" applications or "structural" applications.

"Coating" applications may be found in the marine field (corrosion-resistant coating for boats, for example), in the field of metal containers (cans for food use, for example), in the coil coating field, and in the field of coatings for motor vehicles, to name but a few of them.

"Structural" applications may be found in the field of composites (reinforcing fibers based on glass, boron or graphite for example), in the civil engineering field, in the fields of floor coverings, of construction, of electrical laminates (printed circuits), of electrical and electronic applications (transformers and insulators, for example), of adhesives, and of tooling (prototypes and moulds, for example), to name but a few of them.

Epoxy resins are industrially obtained by using epichlorohydrin as starting material and epichlorohydrin is industrially obtained in a prior separate step by dehydrochlorination of dichloropropanol.

International application WO 2005/054167 filed under the name of SOLVAY Société Anonyme discloses the use of dichloropropanol as starting material for producing epoxy resins in one single step. While such process is simple, opportunities for improvement remain for improving the productivity of the process.

The invention therefore relates to a process for manufacturing an epoxy resin by reaction of dichloropropanol with a compound containing at least one active hydrogen atom and with a basic agent, in order to produce the epoxy resin and a salt, in which epichlorohydrin is formed in situ during the reaction in which at least part of the epichlorohydrin formed in-situ is recovered and at least one fraction of the recovered epichlorohydrin is recycled to the reation, and wherein the chemical formula of the epoxy resin contains at least one 2,3-epoxypropyloxy group.

One of the essential features of the present invention lies in the in situ formation of epichlorohydrin during the reaction to produce the epoxy resin. It has indeed surprisingly been found that the in situ formation of epichlorohydrin allows among others to reduce the molecular weight of the epoxy resin obtained, and/or to reduce the viscosity of the epoxy resin obtained and/or to reduce the amount of organic chlorine found in the said epoxy resin.

In the process according to the invention, at least one part of the epichlorohydrin formed in situ can be recovered. At least one part of the epichlorohydrin formed in situ is preferably recovered.

In the process according to the invention, at least one fraction of the recovered epichlorohydrin can be recycled to the reaction. At least one fraction of the recovered epichlorohydrin is preferably recycled to the reaction.

In the process according to the invention, it is preferred that at least one part of the epichlorohydrin formed in situ is recovered and that at least one fraction of the recovered epichlorohydrin is recycled to the reaction.

The recycling of epichlorohydrin provides the further advantage of a reaction medium less concentrated in the salt, in the absence of an external solvent, reducing thereby the complexity of the process (less precipitates, easier homogenization by stirring, *etc.*).

That process could compete with the classical process while avoiding investing in a separate industrial production of epichlorohydrin, sparing the associated investment costs.

In the process according to the invention, the expression "epoxy resin" is understood to mean a monomer or a polymer, the chemical formula of which contains at least one 2,3-epoxypropyloxy group. The epoxy resin is preferably a polymer.

Examples of monomers are for instance, monoglycidyl ethers, polyglycidyl ethers, or a mixture thereof. The polyglycidyl ether can be a diglycidyl ether, like the diglycidyl ether of bisphenol A (DGEBA) or the diglycidyl ether of isosorbide (diglycidylether of 1,4:3,6-dianhydro-D-sorbitol) for instance.

The term "polymer" is understood to mean molecules comprising several units joined to one another by covalent bonds, often in a repeating manner, these units being referred to as repeating units. The number of repeating units is greater than zero. A polymer contains at least one type of repeating unit. When the polymer contains only a single type of repeating unit, it is known as a homopolymer. When the polymer contains more than a single type of repeating unit, it is known as a copolymer. The copolymer may be of statistical, alternating or block type, as described in "Polymer Science Dictionary, M.S.M., Elsevier Applied Science, London and New York, 1989, page 86".

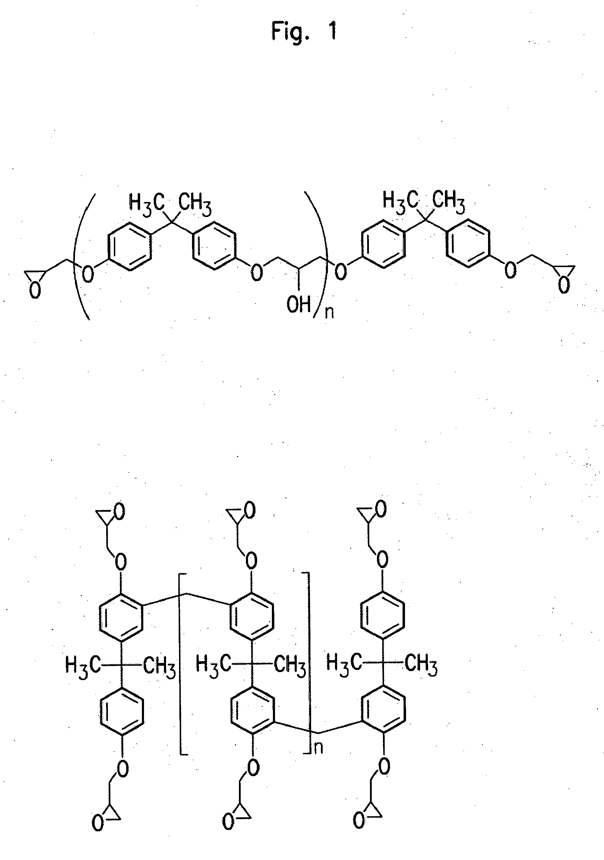

Examples of chemical formulae of epoxy resins are presented in Figure 1, when n is higher than or equal to zero and preferably higher than zero.

In the process according to the invention, the epoxy resin is preferably selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof.

In the process according to the invention, the epoxy resin is more preferably a liquid epoxy resin. The expression "liquid epoxy resin" is understood to mean Type I Grade 1 Classes A and B, Type II Grade 1 Classes A, B and C, Type IV Grade 1 Classes A to D, Type V Grade 1 Classes A and B and Type VI Grade 1 Class A resins, as defined in the ASTM D 1763 - 00 (2005) standard entitled "Standard Specifications for Epoxy Resins".

In the process according to the invention, by dichloropropanol, one intends to denote anyone of 1,3-dichloropropane-2-ol, 2,3-dichloropropane-1-ol, and mixture thereof. Mixtures consisting essentially of 1,3-dichloropropane-2-ol and 2,3-dichloropropane-1-ol are preferred. In the mixture of 1,3-dichloropropane-2-ol and 2,3-dichloropropane-1-ol, the 1,3-dichloro-2-propanol content with respect to the total content of the two dichloropropanol isomers is preferably higher than or equal to 100 g/kg, yet preferably higher than or equal to 300 g/kg, still preferably higher than or equal to at least 400 g/kg, more preferably higher than or equal to 750 g/kg, still more preferably higher than or equal to 800 g/kg, yet more preferably higher than or equal to 900 g/kg, and most preferably higher than or equal to 920 g/kg. This content of 1,3-dichloro-2-propanol in the mixture is generally at most 990 g/kg and usually at most 960 g/kg. Contents of 925, 930, 935, 940, 945, 950 or 955 g/kg are particularly convenient. It is also possible to use a dichloropropanol composed essentially of 1,3-dichloro-2-propanol.

In the process according to the invention, the dichloropropanol may be derived from several processes such as, for example, the allyl chloride hypochlorination process, the allyl alcohol chlorination process, the glycerol hydrochlorination process, the glycerol monochlorohydrin hydrochlorination process, the epichlorohydrin hydrochlorination process, the 2,3-dichloropropionaldehyde hydrogenation process as described in documents WO 1997/48667, US 6,350,922 and US 5,744,655, the 1,2-dichlorethylene hydroformylation process as described in document WO 2005/116004, the 1,3-dichloroacetone hydrogenation process as described in documents WO 2005/097722 and WO 2003/064357. 2,3-dichloropropionaldehyde may itself be obtained by chlorination of acrolein and/or hydroformylation of 1,2-dichloroethylene as described in documents US 2,860,146 and WO 2005/116004. 1,3-dichloroacetone may itself be obtained by chlorination of acetone and/or by bromine/chlorine exchange starting from 1,3-dibromoacetone as described in Applications WO 2005/097722 and WO 2005/115954. Acrolein may be obtained by selective oxidation of propylene. 1,2-dichloroethylene may be a by-product of the synthesis of vinyl chloride starting from ethane and/or be obtained by chlorination of acetylene. Acetylene may be obtained by conventional processes such as hydrolysis of calcium carbide and/or pyrolysis of hydrocarbons, crude oil and even coal, such as described in "Industrial Organic Chemistry, Third, Completely Revised Edition, VCH, 1997, pp. 93-98". 1,3-dibromoacetone may be obtained by bromination of acetone, as described in document WO 2005/115954. Acetone may itself be obtained by conventional processes, such as, for example, oxidation of propylene, dehydrogenation of isopropanol and/or decomposition of cumene hydroperoxide, as described in "Industrial Organic Chemistry, Third, Completely Revised Edition, VCH, 1997, pp. 276-277 and 347-355. Other relevant processes are disclosed in US-A-3138618, CN-A-101519489, US-A-859,456 and EP-A-0082572.

In the process according to the invention, at least one part of the dichloropropanol is preferably obtained by reaction between glycerol and a chlorinating agent and/or by reaction between allyl chloride and a hypochlorinating agent and/or by reaction between allyl alcohol and a chlorinating agent and/or by reaction between 2,3-dichloropropionaldehyde and a hydrogenating agent and/or by reaction between 1,2-dichloroethylene and a hydroformylating agent and/or by reaction between 1,3-dichloroacetone and a hydrogenating agent.

In the process according to the invention, the dichloropropanol is more preferably obtained by reaction between glycerol and a chlorinating agent and/or by reaction between allyl chloride and a hypochlorinating agent, and still more preferably by reaction between glycerol and a chlorinating agent as described in Patent Applications WO 2005/054167, WO 2006/100311, WO 2006/100312, WO 2006/100313, WO 2006/100314, WO 2006/100315, WO 2006/100316, WO 2006/100317, WO 2006/106153, WO 2007/054505, WO 2006/100318, WO 2006/100319, WO 2006/100320, WO 2006/106154, WO 2006/106155, WO 2007/144335, WO 2008/107468, WO 2008/101866, WO 2008/145729, WO 2008/110588, WO 2009/000773, WO 2009/043796, WO 2009/121853, WO 2009/077528, WO 2010/066660, WO 2010/029039 and WO 2010/029153, filed in the name of SOLVAY, the contents of which are incorporated herein by reference.

In the process according to the invention, when at least one part of the dichloropropanol is obtained by reaction between glycerol and a chlorinating agent, the chlorinating agent preferably contains hydrogen chloride such as for example described in Patent Application WO 2005/054167 by Solvay SA. The hydrogen chloride may be in the form of a gas or of an aqueous solution of hydrogen chloride or of a mixture of the two, preferably in the form of a gas or of a mixture of a gas and of an aqueous solution of hydrogen chloride. Glycerol may be obtained from fossil or renewable raw materials. It is preferred to use glycerol obtained from renewable materials. A glycerol which is particularly suitable may be obtained during the conversions of fats or oils of vegetable or animal origin, such as saponification, transesterification or hydrolysis reactions. A particularly suitable glycerol may be obtained during the conversion of animal fats. Another particularly suitable glycerol may be obtained during the manufacture of biodiesel. Another particularly suitable glycerol may be obtained during the fatty acid manufacture. Another particularly suitable glycerol may be obtained during the fatty alcohols manufacture.

In the process according to the invention, the dichloropropanol has preferably been obtained by hydrochlorination of glycerol obtained from renewable raw materials.

In the process according to the invention, the dichloropropanol exhibits usually a purity higher than 90 % by weight, preferably higher than or equal to 95 % by weight, more preferably higher than or equal to 99 % by weight and most preferably higher than or equal to 99.9 % by weight.

In the process according to the invention, the compound containing at least one active hydrogen atom can be of any type. This compound may have been obtained from renewable raw materials, fossil raw materials and any combination thereof. This compound is preferably obtained from renewable raw materials.

The compound containing at least one active hydrogen atom is preferably selected from the group consisting of a polyol, a polycarboxylic acid, a polyamine, an amino alcohol, a polyimide, a polyamide, a polyaminoamide, a polyimine, an acid mono- or polyphenol and mixtures of at least two of these compounds.

In the process according to the invention, the compound containing at least one active hydrogen atom is more preferably a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-α-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol.

In the process according to the invention, the compound containing at least one active hydrogen atom is also more preferably a polyol, preferably containing more than three carbon atoms, selected from the group consisting of a polyphenol, a sugar, a polyol derived from a sugar, an acid polyphenol, any derivative thereof, and any mixture thereof. The polyol may be as described in international application EP2011/066689 filed in the name of SOLVAY S.A., the content of which is incorporated herein by reference, more specifically the passages from page 12, line 5, to page 16, line 13.

In the process according to the invention, when the polyol is a product derived from a sugar, the product derived from a sugar is preferably selected from the group consisting of an anhydrosugar, a reduction product from sugar, a reduction product of hydroxymethylfurfural, a difuran derivative of furfural and any mixture thereof.

In the process according to the invention, when the product derived from a sugar is an anhydrosugar, it is preferably selected from the group consisting of isosorbide, isomannide, isoidide and any mixture thereof. The anhydrosugar is more preferably isosorbide.

In the process according to the invention, the basic compound may be an organic or inorganic basic compound. Organic basic compounds are for example amines, phosphines and ammonium, phosphonium or arsonium hydroxides. Inorganic basic compounds are preferred. The expression "inorganic compounds" is understood to mean compounds which do not contain a carbon-hydrogen bond. The inorganic basic compound is preferably chosen from alkali and alkaline-earth metal oxides, hydroxides, carbonates, hydrogencarbonates, phosphates, hydrogenphosphates and borates, and any mixture of at least two of them.

In the process according to the invention, the basic agent is preferably selected from the group consisting of alkali and alkaline-earth metal oxides, hydroxides, carbonates, hydrogen carbonates, and mixtures of at least two of them, and wherein the salt is selected from the group consisting of alkali and alkaline-earth metal chlorides and mixtures of at least two of them. Alkali and alkaline-earth metal oxides and hydroxides are preferred.

In the process according to the invention, the basic compound may be in the form of a liquid, an essentially anhydrous solid, a hydrated solid, an aqueous and/or organic solution or an aqueous and/or organic suspension. The basic compound is preferably in the form of an essentially anhydrous solid, a hydrated solid, an aqueous solution or an aqueous suspension.

The expression "essentially anhydrous solid" is understood to mean a solid of which the water content is less than or equal to 20 g/kg, preferably less than or equal to 10 g/kg and more preferably less than or equal to 1 g/kg.

The expression "hydrated solid" is understood to mean a solid of which the water content is at least 20 g/kg and at most 700 g/kg, preferably at least 50 g/kg and at most 650 g/kg and most particularly preferably at least 130 g/kg and at most 630 g/kg. The hydrates which denote solid combinations of substances with one or more water molecules are examples of hydrated solids.

When the basic compound is used in the form of an aqueous solution, its content in the aqueous solution is generally greater than 20 g/kg, preferably greater than or equal to 70 g/kg and more preferably greater than or equal to 150 g/kg. This content is generally less than or equal to the solubility of the basic solid in water at the reaction temperature.

When the basic compound is used in the form of an aqueous suspension, its content in the aqueous suspension is generally greater than the solubility of the basic solid in water at the reaction temperature, preferably greater than or equal to 20 g/kg and more preferably greater than or equal to 70 g/kg. This content is generally less than or equal to 400 g/kg, preferably less than 300 g/kg.

The preferred basic compounds are in the form of concentrated aqueous solutions or suspensions of sodium hydroxide or calcium hydroxide or in the form of purified caustic brine.

The sodium hydroxide content of solutions or suspensions of sodium hydroxide is generally greater than or equal to 30 g/kg, usually greater than or equal to 40 g/kg, particularly greater than or equal to 60 g/kg, in many cases greater than or equal to 100 g/kg, and preferably greater than or equal to 120 g/kg. This sodium hydroxide content is generally less than or equal to 300 g/kg, commonly less than or equal to 250 g/kg, often less than or equal to 200 g/k and advantageously less than or equal to 160 g/kg. Contents of 125, 130, 135, 140, 145, 150 and 155 g/kg are particularly convenient.

The expression "purified caustic brine" here means sodium hydroxide which contains sodium chloride such as, for example, that produced in a diaphragm electrolysis process. The sodium hydroxide content of the purified caustic brine is generally greater than or equal to 30 g/kg, preferably greater than or equal to 40 g/kg and more preferably greater than or equal to 60 g/kg. This sodium hydroxide content is generally less than or equal to 300 g/kg, preferably less than or equal to 250 g/kg and more preferably less than or equal to 200 g/kg. The sodium chloride content of the purified caustic brine is generally greater than or equal to 30 g/kg, preferably greater than or equal to 50 g/kg and more preferably greater than or equal to 70 g/kg. This sodium chloride content is generally less than or equal to 250 g/kg, preferably less than or equal to 200 g/kg and more preferably less than or equal to 180 g/kg.

It is also possible to use a mixture of several basic agents as a function of the availabilities and of the economic optimization of the industrial site where the process according to the invention is established. The basic agents preferred for producing these mixtures are limewater and solutions of sodium hydroxide and of purified caustic brine, for example, a mixture of limewater and a sodium hydroxide solution, a mixture of limewater and purified caustic brine. These mixtures may be produced in any relative proportion of at least two of these basic agents.

In the process according to the invention, the salt may be chosen from the group consisting of alkali or alkaline-earth metal halides, sulphates, hydrogen sulphates, hydroxides, carbonates, hydrogen carbonates, phosphates, hydrogen phosphates, borates, and mixtures thereof. Alkali and alkaline-earth metal halides are preferred. Alkali and alkaline-earth metal chlorides are more preferred. Sodium and potassium chlorides are still more preferred and sodium chloride is most particularly preferred.

In the process according to the invention, by epichlorohydrin formed in situ, one intends to denote epichlorohydrin which is formed during the reaction and which is sufficiently stable to accumulate in the reaction medium. This epichlorohydrin can be detected in the reaction medium and possibly isolated from said reaction mixture. The detection can be carried out by any type of analysis, like gas chromatography for instance. The isolation can be carried out by any separation method like evaporation for instance. Epichlorohydrin which is consumed as soon as it formed does not comply with the previous requirements for epichlorohydrin formed in situ.

The process according to the invention may comprise adding to the said process epichlorohydrin which has been obtained in another process. Said epichlorohydrin is preferably added to the reaction step of the process according to the invention, more preferably together with the dichloropropanol.

The epichlorohydrin manufactured by any process may originate, for example, from a process for dehydrochlorination of dichloropropanol, for example via a basic compound, from an allyl chloride epoxidation process, or from a combination of the two processes. Among the allyl chloride epoxidation processes, the process for epoxidation via hydrogen peroxide is preferred. It is preferred that at least one part of the epichlorohydrin is obtained by dehydrochlorination of dichloropropanol, preferably by dehydrochlorination of dichloropropanol with a basic compound.

In the process according to the invention, when present, at least one portion of the epichlorohydrin obtained in another process is preferably obtained by reaction between dichloropropanol and at least one basic compound.

In the process according to the invention, when the epichlorohydrin obtained in another process has been obtained by dehydrochlorination of dichloropropanol, said epichlorohydrin is preferably obtained in a process as the major product.

In the process according to the invention, at least one part of the epichlorohydrin formed in situ can be recovered. At least one fraction of the recovered epichlorohydrin can be recycled to the reaction.

In the process according to the invention, it is preferred that at least one part of the epichlorohydrin formed in situ is recovered and that at least one fraction of the recovered epichlorohydrin is recycled to the reaction.

In the process according to the invention, preferably at least 50 % by weight, more preferably 90 % by weight, still more preferably 95 % by weight and most preferably 99 % by weight of the epichlorohydrin formed in situ is recovered. This percentage is preferably of at most 99.9 % by weight.

In the process according to the invention, preferably at least 50 % by weight, more preferably 90 % by weight, still more preferably 95 % by weight and most preferably 99 % by weight of the recovered epichlorohydrin is recycled to the reaction. This percentage is preferably of at most 99.9 % by weight.

In the process according to the invention, the ratio between the epichlorohydrin formed in situ that is recovered and recycled to the reaction and the epichlorohydrin obtained in another process and added to the reaction is usually higher than or equal to 10 % by weight, preferably higher than or equal to 50 % by weight, more higher than or equal to 90 % by weight, and most higher than or equal to 99 % by weight. This ratio is usually lower than 99.9 by weight. A process where substantially no epichlorohydrin obtained in another process is added to the reaction is particularly convenient.

In the process according to the invention, the epichlorohydrin formed in situ can be recovered by any appropriate separation method or combination of separation methods. A process comprising at least one liquid-liquid phase separation step, in particular to remove at least one part of the epichlorohydrin formed in situ, is convenient.

In the process according to the invention, the reaction can be carried out in the presence of a solvent. Epichlorohydrin is not considered as solvent. The solvent can be selected from the group consisting of alcohols, ketones, aliphatic nitriles, ethylene glycol ethers, mixtures thereof and the like.

In the process according to the invention, at least one of the dichloropropanol, of the compound containing at least one active hydrogen atom, of the epichlorohydrin obtained in the other process, of the basic agent and of the solvent, is preferably obtained from renewable raw materials.

In the process according to the invention, it is more preferred that at least one of the dichloropropanol, of the compound containing at least one active hydrogen atom, of the basic agent, of the possible solvent and of the epichlorohydrin obtained in the other process is obtained from renewable raw materials.

In the process according to the invention, the reaction is usually carried out in a reaction zone. The expression "reaction zone" is understood to mean a zone where at least, dichloropropanol, the compound containing at least one active hydrogen atom and the basic compound, are present. A reaction solvent may be optionally present in the reaction zone.

The process according to the invention can be carried out in a discontinuous or continuous or semi-continuous mode. By continuous mode, one intends to denote a mode wherein the compound containing at least one active hydrogen atom, the dichloropropanol, and the basic agent are continuously fed to the reaction zone and wherein the epoxy resin is continuously withdrawn from the process. By discontinuous mode, one intends to denote any other mode. The semi-continuous mode can be considered as a discontinuous mode. By the term "continuously", one intends to denote without substantial interruption. An interruption is considered as substantial when the duration of the interruption of the considered feed or withdrawal stream amounts for more than 5 %, preferably 10 % and more preferably 15 % of the total feed or withdrawal duration. The discontinuous mode of operation is preferred.

In the process according to the invention, the ratio of the quantity of dichloropropanol expressed in mol of dichloropropanol and of the quantity of the compound containing at least one active hydrogen atom expressed as mol of active hydrogen atoms, fed to the reaction zone, is usually higher than or equal to 0.1, frequently higher than or equal to 0.3, often higher than or equal to 0.6, in particular higher than or equal to 0.9 and more particularly higher than 0.95. This ratio is usually lower than or equal to 10, frequently lower than or equal to 8, often lower or equal to 5, in particular lower than or equal to 3, more particularly lower or equal to 2 and even more particularly lower or equal to 1.5.

In a particular variant of the process according to the invention, the ratio of the quantity of dichloropropanol expressed in mol of dichloropropanol and of the quantity of the compound containing at least one active hydrogen atom expressed as mol of active hydrogen atoms, fed to the reaction zone, is preferably higher than 1, more preferably higher than or equal to 2, still more preferably higher than or equal to 3, yet more preferably than or equal to 5 and more particularly higher than 10. This ratio is preferably lower than or equal to 100, more preferably lower than or equal to 50, still more preferably lower or equal to 40, yet more preferably lower than or equal to 30, and most preferably lower or equal to 20. In this variant the invention relates to a process for manufacturing an epoxy resin and epichlorohydrin by reaction of dichloropropanol, with a compound containing at least one active hydrogen atom, and a basic agent, in order to produce the epoxy resin, epichlorohydrin and a salt, in which epichlorohydrin is formed in situ during the reaction.

In the process according to the invention, the ratio of the quantity of the recycled epichlorohydrin expressed in mol of epichlorohydrin and the quantity of dichloropropanol expressed in mol of dichloropropanol fed to the reaction zone, is usually higher than or equal to 0.01, generally higher than or equal to 0.1, frequently higher than or equal to 0.3, often higher than or equal to 0.5, in many cases higher than or equal to 0.7 and in particular higher than or equal to 0.9. This ratio is preferably lower than or equal to 0.999, more preferably lower than or equal to 0.995, and in particular lower than or equal to 0.99.

In the process according to the invention, when the reaction is carried out in a reaction zone, said reaction zone is fed with the dichloropropanol, with the compound containing at least one active hydrogen atom, with the basic agent and with the recycled epichlorohydrin, and the process exhibits preferably exhibits at least one of the following features :
- the ratio of the quantity of dichloropropanol expressed in mol of dichloropropanol and of the quantity of the compound containing at least one active hydrogen atom expressed as mol of active hydrogen atoms, fed to the reaction zone, is higher than or equal to 0.1 and lower than or equal to 10,
- the ratio of the quantity of the epichlorohydrin expressed in mol of recycled epichlorohydrin and the quantity of dichloropropanol expressed in mol of dichloropropanol, fed to the reaction zone, is higher than or equal to 0.01 and lower than or equal to 0.999.

In the process according to the invention, the reaction is usually carried out at a temperature higher than or equal to 0°C, often higher than or equal to 10°C, frequently higher than or equal to 20°C and in particular higher than or equal to 30°C. That temperature is generally lower than or equal to 140°C, often lower than or equal to 120°C, frequently lower than or equal to 110°C and in particular lower than or equal to 100°C.

In the process according to the invention, the reaction is usually carried out at a pressure higher than or equal to 0.01 bar absolute, often higher than or equal to 0.02 bar absolute, frequently higher than or equal to 0.04 bar absolute and in particular higher than or equal to 0.1 bar absolute. That pressure is generally lower than or equal to 5 bar absolute, often lower than or equal to 3 bar absolute, frequently lower than or equal to 2 bar absolute and in particular lower than or equal to 1.1 bar absolute.

When the process according to the invention is carried out in the continuous mode, the residence time, defined by the volume of the reaction zone divided by the sum of the volumes flow rates of dichloropropanol and of possibly epichlorohydrin, fed to the reaction zone is higher than or equal to 0.5 h, often higher than or equal to 1 h, frequently higher than or equal to 2 h and in particular higher than or equal to 3 h. That residence time is generally lower than or equal to 24 h, often lower than or equal to 15 h, frequently lower than or equal to 10 h and in particular lower than or equal to 7 h.

When the process according to the invention is carried out in the discontinuous mode, the reaction time is generally higher than or equal to 1 h, often higher than or equal to 2 h, frequently higher than or equal to 3 h and in particular higher than or equal to 4 h. That reaction time is generally lower than or equal to 24 h, often lower than or equal to 20 h, frequently lower than or equal to 15 h and in particular lower than or equal to 12 h.

In the process according to the invention, when the basic agent is sodium hydroxide and the salt is sodium chloride obtained as a brine, the process preferably comprises treating the brine to reduce its Total Organic Carbon, electrolyzing the treated brine to produce hydrogen, chlorine and the caustic soda, reacting the hydrogen and the chlorine to produce hydrogen chloride, and reacting the hydrogen chloride with glycerol to produce the dichloropropanol. In that case, it is preferred that at least one part of the hydrogen chloride has been obtained by reacting hydrogen and chlorine, in a molar ratio between hydrogen and chlorine lower than or equal to 1. In that case, it is also preferred that the molar ratio between the hydrogen chloride consumed in the process, and the sodium chloride generated in the process, expressed as elemental chlorine (Cl) is higher than or equal to 0.9. In that case, it is most preferred that at least one part of the hydrogen chloride has been obtained by reacting hydrogen and chlorine, in a molar ratio between hydrogen and chlorine lower than or equal to 1, and that the molar ratio between the hydrogen chloride consumed in the process, and the sodium chloride generated in the process, expressed as elemental chlorine (Cl) is higher than or equal to 0.9.

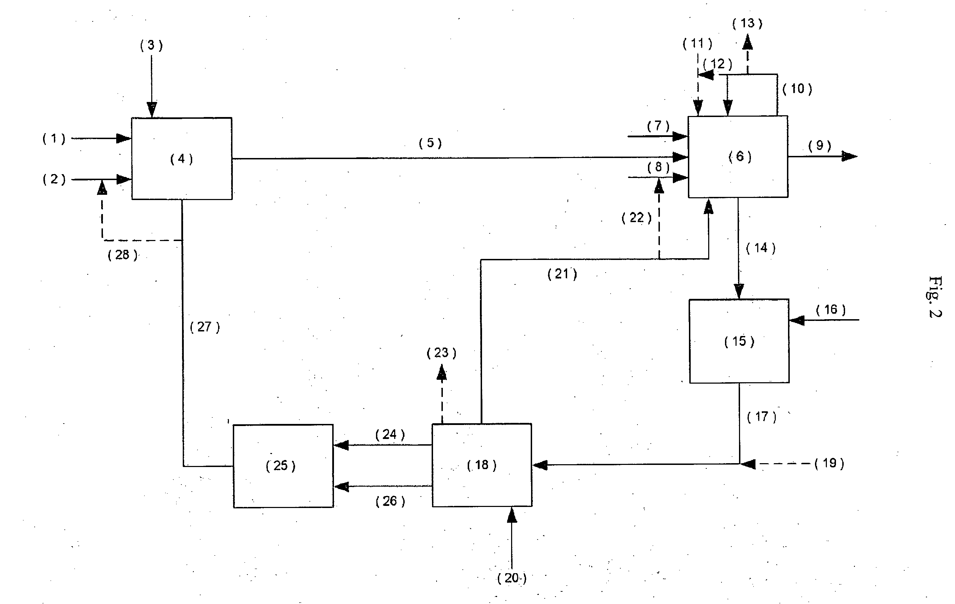

Figure 2 represents a non-limiting embodiment of the process according to the present invention.

A first stream of glycerol, preferably obtained from renewable raw materials, is fed to a first vessel (4) via a first line (1). A second stream of hydrogen chloride, gaseous, as an aqueous solution or a mixture thereof, is fed to first vessel (4) via a second line (2). A third stream of a catalyst, preferably a carboxylic acid is fed to first vessel (4) via a third line (3). First vessel (4) may comprise any combination of reactors and separation equipment. A fourth stream of dichloropropanol is withdrawn from first vessel (4) via a fourth line (5) and is fed to a second vessel (6). Also introduced in second vessel (6) are a fifth stream of a compound containing at least one active hydrogen atom via a fifth line (7) and a sixth stream of sodium hydroxide via a sixth line (8). A seventh stream of epoxy resin is withdrawn from second vessel (6) via a seventh line (9) which may be sent to storage, to further processing such as purification, or to other equipment for further reaction. An eight stream of epichlorohydrin formed in situ in second vessel (6) is withdrawn from second vessel (6) and recycled to second vessel (6) via an eighth line (10). A ninth stream of epichlorohydrin not formed in second vessel (6) may also be fed to second vessel (6) via a ninth line (11). Part of the eighth stream of epichlorohydrin may be added as a tenth stream to the ninth stream of epichlorohydrin via a tenth line (12). Part of the eight stream of the recycled epichlorohydrin may be withdrawn as an eleventh stream via a eleventh line (13) and may be sent to storage, to further processing such as purification, or to other equipment for further reaction. A twelfth stream of brine is withdrawn from second vessel (6) via a twelfth line (14) and fed to a third vessel (15). Third vessel (15) is also fed with a thirtheenth stream containing an oxidant, preferably "active chlorine" via a thirtheenth line (16). The expression "active chlorine" is understood to mean molecular chlorine and its reaction products with water, chloride ions or with a basic agent, such as hypochlorous acid, trichloride ion and sodium hypochlorite for example. A fourtheenth stream of brine is withdrawn from third vessel (16) via a fourtheenth line (17) and fed to a fourth vessel (18). A fifteenth stream of brine may be supplied via a fifteenth line (19). Also supplied to fourth vessel (18) is a sixteenth stream of electricity via a sixteenth line (20). A seventeenth stream of sodium hydroxide is withdrawn from fourth vessel (18) via a seventeenth line (21) and fed to second vessel (6). Part of the seventeenth stream of sodium hydroxide may be added as an eighteenth stream to the sixth stream of sodium hydroxide via a eighteenth line (22). A part of the seventeenth stream of sodium hydroxide may be withdrawn as a nineteenth stream via a nineteenth line (23). A twentieth stream of hydrogen is withdrawn from fourth vessel (18) via a twentieth line (24) and fed to a fifth vessel (25). Also withdrawn from fourth vessel (18) and fed to fifth vessel (25) is a twenty-first stream of chlorine via an twenty-first line (26). A twenty-second stream of hydrogen chloride is withdrawn from fifth vessel (25) and fed to first vessel (4) via a twenty-second line (27). Part of thetwenty-second stream of hydrogen chloride may be added to the second stream of hydrogen chloride as a twenty-third stream via a twenty-third line (28).

Vessels (4), (6), (15), (18) and (25) contain reaction vessel of any well-known suitable type, including for example, one or more discontinuous or continuous reactors, and separation vessel of any well-known suitable type, including for example, one or more distillation columns, evaporators, strippers, settlers, adsorbers, absorbers, ion exchange equipments or combination thereof.

More specifically, vessel (18) contains any type of well-known electrolysis suitable cells, including for example, mercury, diaphragm and membrane chlor-alkali electrolysis cells.

## Claims

1. Process for manufacturing an epoxy resin by reaction of dichloropropanol with a compound containing at least one active hydrogen atom and with a basic agent, in order to produce the epoxy resin and a salt, in which epichlorohydrin is formed in situ during the reaction, in which at least one part of the epichlorohydrin formed in situ is recovered and at least one fraction of the recovered epichlorohydrin is recycled to the reaction, and wherein the chemical formula of the epoxy resin contains at least one 2,3-epoxypropyloxy group.

2. Process according to claim 1 wherein the epoxy resin is selected from the group consisting of Type I Grade 1 Classes A to H, Type II Grade 1 Classes A to F and Type VI Grade 1 Class A resins, as defined in the ASTM D 1763 - 00 (2005) standard entitled "Standard Specifications for Epoxy Resins", and any mixture thereof, and wherein the epoxy resin is preferably a liquid epoxy resin selected from the group consisting of Type I Grade 1 Classes A and B, Type II Grade 1 Classes A, B and C and Type VI Grade 1 Class A resins, as defined in the ASTM D 1763 - 00 (2005) standard entitled "Standard Specifications for Epoxy Resins", and any mixture thereof.

3. Process according to claim 1 or 2 wherein the compound containing at least one active hydrogen atom is a polyol selected from the group constituted of bisphenol A (4,4'-dihydroxy-2,2-diphenylpropane, 4,4'-isopropylidenediphenol), tetrabromobisphenol A (4,4'-isopropylidenebis(2,6-dibromophenol)), bisphenol AF (4,4'-[2,2,2-trifluoro-1-(trifluoromethyl)ethylidene]bisphenol), hexafluorobisphenol A (4,4'-dihydroxy-2,2-diphenyl-1,1,1,3,3,3-hexafluoropropane), 1,1,2,2-tetra(p-hydroxyphenyl)ethane, tetramethylbisphenol (4,4'-dihydroxy-3,3',5,5'-tetramethyl bisphenol), 1,5-dihydroxynaphthalene, 1,1',7,7'-tetrahydroxydinaphthylmethane, 4,4'-dihydroxy-α-methylstilbene, a condensation product of bisphenol A with formaldehyde (bisphenol A novolac), a condensation product of phenol with formaldehyde, preferably bisphenol F (mixture of o,o', o,p' and p,p' isomers of dihydroxydiphenylmethane), a condensation product of cresol with formaldehyde (mixture of o,o', o,p' and p,p' isomers of methylhydroxydiphenylmethane), an alkylation product of phenol and of dicyclopentadiene (2,5-bis[hydroxyphenyl]octahydro-4,7-methano-5H-indene), a condensation product of phenol and of glyoxal (tetrakis(4-hydroxyphenyl)ethane), a condensation product of phenol and of a hydroxybenzaldehyde (e.g. tris(4-hydroxyphenyl)methane), 1,1,3-tris(p-hydroxyphenyl)propane, and mixtures of at least two of them, or p-aminophenol, or wherein the compound containing at least one active hydrogen atom is a polyol selected from the group consisting of a polyphenol, a sugar, preferably a sugar selected from the group constituted of isosorbide, isomannide, isoidide and any mixture thereof, a polyol derived from a sugar, an acid polyphenol, any derivative thereof, and any mixture thereof.

4. Process according to any one of claims 1 to 3, wherein the basic agent is selected from the group consisting of alkali and alkaline-earth metal oxides, hydroxides, carbonates, hydrogen carbonates, and mixtures of at least two of them, and wherein the salt is selected from the group consisting of alkali and alkaline-earth metal chlorides, and mixtures of at least two of them.

5. Process according to any one of claims 1 to 4, comprising at least one liquid-liquid phase separation step to recover at least one part of the epichlorohydrin formed in situ.

6. Process according to any one of claims 1 to 5, wherein at least 50 % of the recovered epichlorohydrin is recycled to the reaction.

7. Process according to any one of claims 1 to 6, comprising adding to the reaction, epichlorohydrin which has been obtained in another process.

8. Process according to any one of claims 1 to 7, wherein the reaction is carried out in the presence of a solvent.

9. Process according to claim 8, wherein at least one of the dichloropropanol, of the compound containing at least one active hydrogen atom, of the epichlorohydrin obtained in the other process, of the basic agent and of the solvent, has been obtained from renewable raw materials.

10. Process according to any one of claims 1 to 9, wherein the reaction is carried out in a reaction zone, said reaction zone is fed with the dichloropropanol, with the compound containing at least one active hydrogen atom, with the basic agent and with the recycled epichlorohydrin, and exhibiting at least one of the following features :
• the ratio between the quantity of dichloropropanol expressed in mol of dichloropropanol and of the quantity of the compound containing at least one active hydrogen atom expressed as mol of active hydrogen atoms, fed to the reaction zone, is higher than or equal to 0.1 and lower than or equal to 10,
• the ratio between the quantity of the recycled epichlorohydrin expressed in mol of epichlorohydrin and the quantity of dichloropropanol expressed in mol of dichloropropanol ; fed to the reaction zone, is higher than or equal to 0.01 and lower than or equal to 0.999.

11. Process according to any one of claims 1 to 10 carried out under discontinuous mode.

12. Process according to any one of claims 1 to 11, wherein the basic agent is caustic soda and the salt is sodium chloride obtained as a brine exhibiting a Total Organic Carbon, comprising treating the brine to reduce its Total Organic Carbon, electrolyzing the treated brine to produce hydrogen, chlorine and the caustic soda, reacting the hydrogen and the chlorine to produce hydrogen chloride, and reacting the hydrogen chloride with glycerol to produce the dichloropropanol.

13. Process according to claim 12, wherein at least one part of the hydrogen chloride has been obtained by reacting hydrogen and chlorine, in a molar ratio between hydrogen and chlorine lower than or equal to 1.

14. Process according to claim 12 or 13, wherein the molar ratio between the hydrogen chloride consumed in the process, and the sodium chloride generated in the process, expressed as elemental chlorine (Cl) is higher than or equal to 0.9.

## Patentansprüche

1. Verfahren zur Herstellung eines Epoxidharzes durch Reaktion von Dichlorpropanol mit einer Verbindung, die mindestens ein aktives Wasserstoffatom enthält, und mit einem basischen Mittel, wobei man das Epoxidharz und ein Salz erhält, wobei während der Reaktion Epichlorhydrin in situ gebildet wird, wobei mindestens ein Teil des in situ gebildeten Epichlorhydrins gewonnen wird und mindestens eine Fraktion des gewonnenen Epichlorhydrins in die Reaktion zurückgeführt wird und wobei die chemische Formel des Epoxidharzes mindestens eine 2,3-Epoxypropyloxygruppe enthält.

2. Verfahren nach Anspruch 1, bei dem das Epoxidharz aus der Gruppe bestehend aus Harzen vom Type I Grade 1 Classes A bis H, vom Type II Grade 1 Classes A bis F und vom Type VI Grade 1 Class A gemäß der in der ASTM-NORM D 1763 - 00 (2005) mit dem Titel "Standard Specifications for Epoxy Resins" angegebenen Definition und einer beliebigen Mischung davon ausgewählt wird und wobei es sich bei dem Epoxidharz vorzugsweise um ein flüssiges Epoxidharz aus der Gruppe bestehend aus Harzen vom Type I Grade 1 Classes A und B, vom Type II Grade 1 Classes A, B und C und vom Type VI Grade 1 Class A gemäß der in der ASTM-NORM D 1763 - 00 (2005) mit dem Titel "Standard Specifications for Epoxy Resins" angegebenen Definition und eine beliebige Mischung davon handelt.

3. Verfahren nach Anspruch 1 oder 2, bei dem es sich bei der Verbindung, die mindestens ein aktives Wasserstoffatom enthält, um ein Polyol aus der Gruppe bestehend aus Bisphenol A (4,4'-Dihydroxy-2,2-diphenylpropan, 4,4'-Isopropylidenediphenol), Tetrabrombisphenol A (4,4'-Isopropylidenbis(2,6-dibromphenol)), Bisphenol AF (4,4'-[2,2,2-Trifluor-1-(trifluormethyl)ethyliden]bisphenol), Hexafluorbisphenol A (4,4'-Dihydroxy-2,2-diphenyl-1,1,1,3,3,3-hexafluorpropan), 1,1,2,2-Tetra(p-hydroxyphenyl)ethan, Tetramethylbisphenol (4,4'-Dihydroxy-3,3',5,5'-tetramethylbisphenol), 1,5-Dihydroxynaphthalin, 1,1',7,7'-Tetrahydroxydinaphthylmethan, 4,4'-Dihydroxy-α-methylstilben, einem Kondensationsprodukt von Bisphenol A mit Formaldehyd (Bisphenol-A-Novolak), einem Kondensationsprodukt von Phenol mit Formaldehyd, vorzugsweise Bisphenol F (Gemisch von o,o'-, o,p'- und p,p'-Isomeren von Dihydroxydiphenylmethan), einem Kondensationsprodukt von Kresol mit Formaldehyd (Gemisch von o,o'-, o,p'- und p,p'-Isomeren von Methylhydroxydiphenylmethan), einem Alkylierungsprodukt von Phenol und Dicyclopentadien (2,5-Bis[hydroxyphenyl]octahydro-4,7-methano-5H-inden), einem Kondensationsprodukt von Phenol und Glyoxal (Tetrakis(4-hydroxyphenyl)ethan), einem Kondensationsprodukt von Phenol und einem Hydroxybenzaldehyd (z.B. Tris(4-hydroxyphenyl)methan), 1,1,3-Tris(p-hydroxyphenyl)propan und Mischungen von mindestens zwei davon oder p-Aminophenol handelt oder wobei es sich bei der Verbindung, die mindestens ein aktives Wasserstoffatom enthält, um ein Polyol aus der Gruppe bestehend aus einem Polyphenol, einem Zucker, vorzugsweise einem Zucker aus der Gruppe bestehend aus Isosorbid, Isomannid, Isoidid und einer beliebigen Mischung davon, einem von einem Zucker abgeleiteten Polyol, einem sauren Polyphenol, einem beliebigen Derivat davon und einer beliebigen Mischung davon handelt.

4. Verfahren nach einem der Ansprüche 1 bis 3, bei dem das basische Mittel aus der Gruppe bestehend aus Alkali- und Erdalkalimetalloxiden, -hydroxiden, -carbonaten, -hydrogencarbonaten und Mischungen von mindestens zwei davon ausgewählt wird und wobei das Salz aus der Gruppe bestehend aus Alkali- und Erdalkalimetallchloriden und Mischungen von mindestens zwei davon ausgewählt wird.

5. Verfahren nach einem der Ansprüche 1 bis 4, das mindestens einen Flüssig-Flüssig-Phasentrennungsschritt zur Gewinnung mindestens eines Teils des in situ gebildeten Epichlorhydrins umfasst.

6. Verfahren nach einem der Ansprüche 1 bis 5, bei dem mindestens 50% des gewonnenen Epichlorhydrins in die Reaktion zurückgeführt werden.

7. Verfahren nach einem der Ansprüche 1 bis 6, bei dem man bei einem anderen Verfahren erhaltenes Epichlorhydrin zu der Reaktion gibt.

8. Verfahren nach einem der Ansprüche 1 bis 7, bei dem die Reaktion in Gegenwart eines Lösungsmittels durchgeführt wird.

9. Verfahren nach Anspruch 8, bei dem das Dichlorpropanol, die Verbindung, die mindestens ein aktives Wasserstoffatom enthält, das in dem anderen Verfahren erhaltene Epichlorhydrin, das basische Mittel und/oder das Lösungsmittel aus nachwachsenden Rohstoffen erhalten worden ist bzw. sind.

10. Verfahren nach einem der Ansprüche 1 bis 9, bei dem die Reaktion in einer Reaktionszone durchgeführt wird, wobei die Reaktionszone mit dem Dichlorpropanol, mit der Verbindung, die mindestens ein aktives Wasserstoffatom enthält, mit dem basischen Mittel und mit dem zugeführten Epichlorhydrin gespeist wird und mindestens eines der folgenden Merkmale aufweist:
• das Verhältnis zwischen der Menge an Dichlorpropanol, ausgedrückt in Mol Dichlorpropanol, und der Menge der Verbindung, die mindestens ein aktives Wasserstoffatom enthält, ausgedrückt als Mol aktive Wasserstoffatome, die der Reaktionszone zugeführt werden, ist größer gleich 0,1 und kleiner gleich 10,
• das Verhältnis zwischen der Menge des zurückgeführten Epichlorhydrins, ausgedrückt in Mol Epichlorhydrin, und der Menge an Dichlorpropanol, ausgedrückt in Mol Dichlorpropanol, die der Reaktionszone zugeführt werden, ist größer gleich 0,01 und kleiner gleich 0,999.

11. Verfahren nach einem der Ansprüche 1 bis 10, das in diskontinuierlichem Modus durchgeführt wird.

12. Verfahren nach einem der Ansprüche 1 bis 11, bei dem es sich bei dem basischen Mittel um Ätznatron handelt und es sich bei dem Salz um Natriumchlorid handelt, das in Form einer Salzlösung mit einem Total Organic Carbon erhalten wird, bei dem man die Salzlösung zur Verringerung ihres Total Organic Carbon behandelt, die behandelte Salzlösung zur Produktion von Wasserstoff, Chlor und dem Ätznatron elektrolysiert, den Wasserstoff und das Chlor zu Chlorwasserstoff umsetzt und den Chlorwasserstoff mit Glycerin zu dem Dichlorpropanol umsetzt.

13. Verfahren nach Anspruch 12, bei dem mindestens ein Teil des Chlorwasserstoffs durch Umsetzung von Wasserstoff und Chlor in einem Molverhältnis zwischen Wasserstoff und Chlor kleiner gleich 1 erhalten worden ist.

14. Verfahren nach Anspruch 12 oder 13, bei dem das Molverhältnis zwischen dem bei dem Verfahren verbrauchten Chlorwasserstoff und dem bei dem Verfahren anfallenden Natriumchlorid, ausgedrückt als elementares Chlor (Cl), größer gleich 0,9 ist.

## Revendications

1. Procédé de fabrication d'une résine époxyde par réaction de dichloropropanol avec un composé contenant au moins un atome d'hydrogène actif et avec un agent basique, afin de produire la résine époxyde et un sel, dans lequel de l'épichlorhydrine est formée in situ pendant la réaction, dans lequel au moins une partie de l'épichlorhydrine formée in situ est récupérée et au moins une fraction de l'épichlorhydrine récupérée est recyclée dans la réaction, et dans lequel la formule chimique de la résine époxyde contient au moins un groupe 2,3-époxypropyloxy.

2. Procédé selon la revendication 1 dans lequel la résine époxyde est choisie dans le groupe constitué par les résines de type I, qualité 1, classes A à H, de type II, qualité 1, classes A à F, et de type VI, qualité 1, classe A, selon la définition de la norme ASTM D 1763-00 (2005) intitulée « Standard Specifications for Epoxy Resins », et tous les mélanges de celles-ci, et dans lequel la résine époxyde est de préférence une résine époxyde liquide choisie dans le groupe constitué par les résines de type I, qualité 1, classes A et B, de type II, qualité 1, classes A, B et C, et de type VI, qualité 1, classe A, selon la définition de la norme ASTM D 1763-00 (2005) intitulée « Standard Specifications for Epoxy Resins », et tous les mélanges de celles-ci.

3. Procédé selon la revendication 1 ou 2 dans lequel le composé contenant au moins un atome d'hydrogène actif est un polyol choisi dans le groupe constitué par le bisphénol A (4,4'-dihydroxy-2,2-diphénylpropane, 4,4'-isopropylidènediphénol), le tétrabromobisphénol A (4,4'-isopropylidènebis(2,6-dibromophénol)), le bisphénol AF (4,4'-[2,2,2-trifluoro-1-(trifluorométhyl)éthylidène]bisphénol), l'hexafluorobisphénol A (4,4'-dihydroxy-2,2-diphényl-1,1,1,3,3,3-hexafluoropropane), le 1,1,2,2-tétra(p-hydroxyphényl)éthane, le tétraméthylbisphénol (4,4'-dihydroxy-3,3',5,5'-tétraméthylbisphénol), le 1,5-dihydroxynaphtalène, le 1,1',7,7'-tétrahydroxydinaphtylméthane, le 4,4'-dihydroxy-α-méthylstilbène, un produit de condensation du bisphénol A avec le formaldéhyde (novolaque de bisphénol A), un produit de condensation de phénol avec le formaldéhyde, de préférence le bisphénol F (mélange d'isomères o,o', o,p' et p,p' de dihydroxy-diphénylméthane), un produit de condensation du crésol avec le formaldéhyde (mélange d'isomères o,o', o,p' et p,p' de méthylhydroxydiphénylméthane), un produit d'alkylation de phénol et de dicyclopentadiène (2,5-bis[hydroxyphényl]octahydro-4,7-méthano-5H-indène), un produit de condensation de phénol et de glyoxal (tétrakis(4-hydroxyphényl)éthane), un produit de condensation de phénol et d'un hydroxybenzaldéhyde (par ex. le tris(4-hydroxyphényl)éthane), le 1,1,3-tris(p-hydroxyphényl)propane, et les mélanges d'au moins deux d'entre eux, ou le p-aminophénol, ou dans lequel le composé contenant au moins un atome d'hydrogène actif est un polyol choisi dans le groupe constitué par un polyphénol, un sucre, de préférence un sucre choisi dans le groupe constitué par l'isosorbide, l'isomannide, l'isoidide et tous les mélanges de ceux-ci, un polyol dérivé d'un sucre, un polyphénol acide, tous les dérivés de ceux-ci, et tous les mélanges de ceux-ci.

4. Procédé selon l'une quelconque des revendications 1 à 3, dans lequel l'agent basique est choisi dans le groupe constitué par les oxydes, hydroxydes, carbonates, hydrogénocarbonates de métaux alcalins et alcalino-terreux, et les mélanges d'au moins deux d'entre eux, et dans lequel le sel est choisi dans le groupe constitué par les chlorures de métaux alcalins et alcalino-terreux, et les mélanges d'au moins deux d'entre eux.

5. Procédé selon l'une quelconque des revendications 1 à 4, comprenant au moins une étape de séparation de phases liquide-liquide pour récupérer au moins une partie de l'épichlorhydrine formée in situ.

6. Procédé selon l'une quelconque des revendications 1 à 5, dans lequel au moins 50 % de l'épichlorhydrine récupérée est recyclée dans la réaction.

7. Procédé selon l'une quelconque des revendications 1 à 6, comprenant l'addition à la réaction, d'épichlorhydrine qui a été obtenue dans un autre procédé.

8. Procédé selon l'une quelconque des revendications 1 à 7, dans lequel la réaction est réalisée en présence d'un solvant.

9. Procédé selon la revendication 8, dans lequel au moins un composant parmi le dichloropropanol, le composé contenant au moins un atome d'hydrogène actif, l'épichlorhydrine obtenue dans l'autre procédé, l'agent basique et le solvant a été obtenu à partir de matières premières renouvelables.

10. Procédé selon l'une quelconque des revendications 1 à 9, dans lequel la réaction est réalisée dans une zone réactionnelle, ladite zone réactionnelle est alimentée avec le dichloropropanol, avec le composé contenant au moins un atome d'hydrogène actif, avec l'agent basique et avec l'épichlorhydrine recyclée, et présentant au moins une des caractéristiques suivantes :
• le rapport entre la quantité de dichloropropanol exprimée en mol de dichloropropanol et la quantité du composé contenant au moins un atome d'hydrogène actif exprimée en mol d'atomes d'hydrogène actifs, introduits dans la zone réactionnelle, est supérieur ou égal à 0,1 et inférieur ou égal à 10,
• le rapport entre la quantité de l'épichlorhydrine recyclée exprimée en mol d'épichlorhydrine et la quantité de dichloropropanol exprimée en mol de dichloropropanol, introduits dans la zone réactionnelle, est supérieur ou égal à 0,01 et inférieur ou égal à 0,999.

11. Procédé selon l'une quelconque des revendications 1 à 10 réalisé en mode discontinu.

12. Procédé selon l'une quelconque des revendications 1 à 11, dans lequel l'agent basique est la soude caustique et le sel est le chlorure de sodium obtenu sous la forme d'une saumure présentant un carbone organique total, comprenant le traitement de la saumure pour réduire son carbone organique total, l'électrolyse de la saumure traitée pour produire de l'hydrogène, du chlore et de la soude caustique, la réaction de l'hydrogène et du chlore pour produire du chlorure d'hydrogène, et la réaction du chlorure d'hydrogène avec du glycérol pour produire le dichloropropanol.

13. Procédé selon la revendication 12, dans lequel au moins une partie du chlorure d'hydrogène a été obtenue en faisant réagir de l'hydrogène et du chlore, dans un rapport molaire entre hydrogène et chlore inférieur ou égal à 1.

14. Procédé selon la revendication 12 ou 13, dans lequel le rapport molaire entre le chlorure d'hydrogène consommé dans le procédé et le chlorure de sodium généré dans le procédé, exprimés en chlore élémentaire (Cl), est supérieur ou égal à 0,9.
